# FASCICULE DE BREVET EUROPEEN

(11) **EP 4 119 104 B1**
(45) Date de publication et mention de la délivrance du brevet: **24.04.2024**
(21) Numéro de dépôt: 22184793.2
(22) Date de dépôt: 13.07.2022
(51) Int. Cl.: A61F 2/66

(54) **SEMELLE POUR LAME FLEXIBLE**
SOHLE FÜR FLEXIBLE LAMELLENFEDER
SOLE FOR FLEXIBLE BLADE

(30) Priorité: 16.07.2021 FR 2107708
(43) Date de publication de la demande: 18.01.2023
(73) Titulaire: Salomon S.A.S., 74370 Epagny Metz-Tessy (FR)
(72) Inventeur: LEICK, Patrick, 74370 VILLAZ (FR); BOUTRIN, Eddy, 74290 MENTHON SAINT BERNARD (FR); ROCHE, Hugo, 07130 TOULAUD (FR)
(74) Mandataire: Lapierre, Stéphane

(56) Documents cités:
- WO-A1-00/27317
- WO-A1-2017/173200
- US-A1- 2017 281 372

## Description

La présente invention concerne une semelle destinée à se fixer sur une lame flexible positionnée entre le corps d'un utilisateur et le sol. Cette lame flexible peut être une prothèse de jambe d'une personne handicapée ou un élément d'une chaussure pour personne valide.

Afin d'améliorer la mobilité des personnes ayant été amputées d'une partie d'une jambe, une grande variété de prothèses sont proposés. Parmi celles-ci, certaines ont été conçues spécifiquement pour une pratique sportive et, notamment, la course à pied. La plupart de ces prothèses se présentent sous la forme d'une lame profilée, généralement en carbone. Ces lames galbées sont fixées au membre amputé, à une première extrémité, et sont en contact avec le sol, à une deuxième extrémité, sensiblement au droit de la première extrémité. Entre les extrémités, la lame suit une courbe convexe, s'étendant vers l'arrière du sportif. Au niveau de la deuxième extrémité de la lame, extrémité destinée à être en contact avec le sol, les fabricants proposent d'ajouter des patins permettant d'améliorer l'adhérence au sol. Souvent ces patins sont directement collés sur la face inférieure de la lame, comme c'est le cas, par exemple, avec des prothèses de Fillauer^{®}. Alternativement, ces patins peuvent être fixés par vis à la lame, comme c'est le cas, par exemple, avec des prothèses de Ottobock^{®}. Cette construction permet de les changer en cas d'usure. Dans les deux cas, ces solutions ne permettent pas un changement rapide de semelage. Pour une pratique de course à pied sur route ou sur piste, ce besoin n'est probablement pas nécessaire. Cependant, lors d'une pratique de course à pied sur terrain variable, comme de la course en montagne ou trail, l'utilisateur pourrait avoir besoin de modifier l'adhérence de son semelage en fonction du terrain sur lequel il va évoluer, notamment si le terrain est boueux, enneigé, on encore accidenté. Il peut ainsi souhaiter changer rapidement son patin, sans utiliser d'outils. Les documents WO2017/173200 et US8535390 proposent deux solutions pour permettre l'interchangeabilité rapide de ces patins. Össur^{®} / Nike^{®} propose, par exemple, de telles semelages. Ces semelles amovibles s'avèrent néanmoins complexes à réaliser.

Par ailleurs, ce type de lame profilée se révèle être moins stable en statique du fait du contact réduit avec le sol. Ces lames sont performantes en dynamique mais peuvent présenter une instabilité au repos ou sur terrain incliné, ce qui nécessite un rééquilibrage fréquent de l'utilisateur qui peut conduire à de la fatigue. Cet inconfort est d'autant plus accentué lorsque la personne est amputée des deux jambes. Pour améliorer le confort, il peut être souhaitable de pouvoir adapter la semelle rapportée pour assurer davantage de portance et de stabilité, en fonction de l'environnement dans lequel évolue l'utilisateur. Les deux semelles amovibles précédentes ne permettent qu'une seule possibilité de portance pour une même semelle.

Le but de l'invention est de proposer une semelle pour lame flexible améliorée.

Un but est notamment de proposer une semelle amovible et facile à mettre en place, sans l'utilisation d'outils.

Un autre but est de proposer une semelle simple à réaliser et économique.

Un autre but est de proposer une semelle compatible avec des accessoires permettant d'améliorer la portance.

L'invention propose une semelle amovible composée d'une partie antérieure comprenant un logement destiné à recevoir une extrémité distale d'une lame flexible, le logement étant délimité par une paroi inférieure, une paroi latérale, une paroi médiale, une paroi frontale et une paroi supérieure, le logement présentant ainsi une ouverture postérieure délimitée par les parois inférieure, latérale, médiale et supérieure de la partie antérieure, l'ouverture postérieure permettant l'insertion de l'extrémité distale de la lame flexible dans le logement.

La semelle amovible est caractérisée par le fait qu'elle comprend une extension postérieure souple prolongeant la paroi inférieure vers l'arrière, l'extension postérieure présentant une face supérieure portant un moyen d'accroche apte à coopérer avec un moyen d'accroche complémentaire disposé sur une face inférieure de la lame flexible.

Cette construction est élémentaire et très facile à réaliser, ce qui la rend également économique. La mise en place de la semelle sur la lame flexible est très simple. Il suffit d'insérer la lame flexible dans le logement de la semelle et de connecter les moyens d'accroche. Pour retirer la semelle, il suffit de réaliser les étapes inverses. L'amovibilité de la semelle permet facilement de changer de semelle en cas d'usure ou si l'on souhaite adapter le semelage au terrain que l'utilisateur va parcourir : plus de grip, plus de crampons... Enfin, grâce à l'extension arrière souple, on peut facilement intercaler une cale entre la semelle et la lame de manière afin d'augmenter la surface de contact de la semelle avec le sol et donc sa stabilité. Ainsi, l'utilisateur peut aisément modifier sa portance lors d'une course sur terrain variable. Il peut, par exemple, augmenter la surface de contact au sol lors des descentes où il aurait besoin de plus stabilité pour améliorer son équilibre. Sa proprioception s'avère également accrue.

Selon des aspects avantageux mais non obligatoires de l'invention, une telle semelle peut incorporer une ou plusieurs des caractéristiques suivantes, prises dans toute combinaison techniquement admissible :
- face supérieure de la paroi inférieure et la face supérieure de l'extension postérieure se prolongent l'une l'autre, formant une surface continue.
- le moyen d'accroche de la face supérieure de l'extension postérieure sont composés de crochets et/ou de boucles.
- la paroi inférieure et l'extension postérieure comprennent des faces inférieures constituées de caoutchouc.
- les parois latérale, médiale et frontale sont en caoutchouc.
- le caoutchouc est caractérisé par une dureté comprise entre 60 et 80 Shore A.
- la paroi inférieure comprend au moins une portion constituée d'un matériau à base de Ethylène-Acétate de Vinyle (EVA) ou mousse PolyUréthane (PU).
- la paroi supérieure est une pièce rapportée.
- la paroi supérieure est réalisée dans un matériau ayant des propriétés anisotropes, moins extensible transversalement que longitudinalement.
- l'extension postérieure présente une largeur sensiblement égale à la largeur de la lame flexible et s'étend sur une longueur supérieure à la largeur de la lame flexible.
- la largeur et la hauteur du logement sont sensiblement égale ou très légèrement supérieure respectivement à la largeur et l'épaisseur de la partie distale de la lame flexible.

L'invention porte également sur :
- un ensemble comprenant une lame flexible présentant une extrémité distale et une face inférieure, la face inférieure portant des moyens d'accroche complémentaires dans une zone arrière, et une semelle amovible telle que définie précédemment, la semelle amovible étant destinée à s'emboiter sur l'extrémité distale de la lame flexible, les moyens d'accroche de la semelle amovible étant apte à coopérer avec les moyens d'accroche complémentaires de la lame flexible.
- l'ensemble décrit précédemment comprenant, en outre, une cale intercalée entre la lame flexible et l'extension postérieure de la semelle amovible, la cale présentant une face inférieure munie de moyens d'accroche complémentaires apte à coopérer avec les moyens d'accroche de la face supérieure de l'extension postérieure de la semelle amovible, la cale présentant une face supérieure munie de moyens d'accroche apte à coopérer avec moyens d'accroche complémentaires de la face inférieure de la lame flexible. Selon une mode de réalisation, la cale comprend au moins une portion constituée d'un matériau à base de Ethylène-Acétate de Vinyle (EVA) ou mousse PolyUréthane (PU). Selon une mode de réalisation, l'extension postérieure de la semelle amovible est apte à recouvrir la surface inférieure de la cale intercalaire au niveau du talon.

D'autre caractéristiques et avantages de l'invention seront mieux compris à l'aide de la description qui va suivre, en regard des dessins annexés illustrant, selon des formes de réalisation non limitatives, comment l'invention peut être réalisée, et dans lequel :
[Fig. 1] La figure 1 est une vue de dessous en perspective avant d'une lame flexible équipée d'une semelle amovible selon un premier mode de réalisation de l'invention.
[Fig. 2] La figure 2 est une vue de dessus en perspective d'une lame flexible et d'une semelle amovible non assemblée à la lame.
[Fig. 3] La figure 3 est une vue en coupe longitudinale médiane, la semelle amovible n'étant pas fixée à la lame.
[Fig. 4] La figure 4 est une vue en coupe longitudinale médiane, la semelle amovible étant fixée à la lame.
[Fig. 5] La figure 5 est une vue de dessus de la semelle amovible.
[Fig. 6] La figure 6 est une vue en coupe longitudinale selon VI-VI de la figure 4.
[Fig. 7] La figure 7 est une vue en coupe longitudinale médiane d'une lame flexible équipée d'une semelle amovible selon un deuxième mode de réalisation de l'invention.
[Fig. 8] La figure 8 est une vue en coupe longitudinale médiane d'une lame flexible équipée d'une semelle amovible selon un troisième mode de réalisation de l'invention.
[Fig. 9] La figure 9 est une vue en coupe longitudinale médiane d'une lame flexible équipée d'une semelle amovible selon un quatrième mode de réalisation de l'invention.
[Fig. 10] La figure 10 est une vue schématique en coupe longitudinale médiane d'une chaussure comprenant une lame flexible équipée d'une semelle amovible.

Ces dessins sont des représentations schématiques des modes de réalisation de l'invention. Il peut y avoir quelques disproportions pour certains éléments constitutifs.

Dans la suite de la description, il sera fait usage de termes tels que « horizontal », « vertical », « transversal », « supérieur », « inférieur », « haut », « bas », « latéral », « médial », « droite », « gauche », « antérieur », « postérieur », « devant », « derrière », « avant », « arrière ». Ces termes doivent être interprétés en fait de façon relative en relation avec la position que la semelle occupe sur un support virtuel équipant un utilisateur en posture normale, et la direction d'avancement normale d'un utilisateur. On considérera la configuration pour laquelle la semelle est posée à plat sur un sol horizontal.

Les termes « latéral » et « médial », de manière conventionnelle, s'entendent comme tournés respectivement vers l'extérieur et vers l'intérieur. Ainsi, le côté médial d'un pied ou d'une semelle est tourné vers le côté médial de l'autre pied ou de l'autre semelle de l'utilisateur.

Le terme « longitudinal » fait référence à une direction talon-orteils correspondant à l'axe X alors que le terme « transversal », fait référence à une direction latéral-médial (pied droit) / médial-latéral (pied gauche) correspondant à l'axe Y et donc sensiblement perpendiculaire à la direction longitudinale. La direction verticale ou bas/haut correspond à l'axe Z.

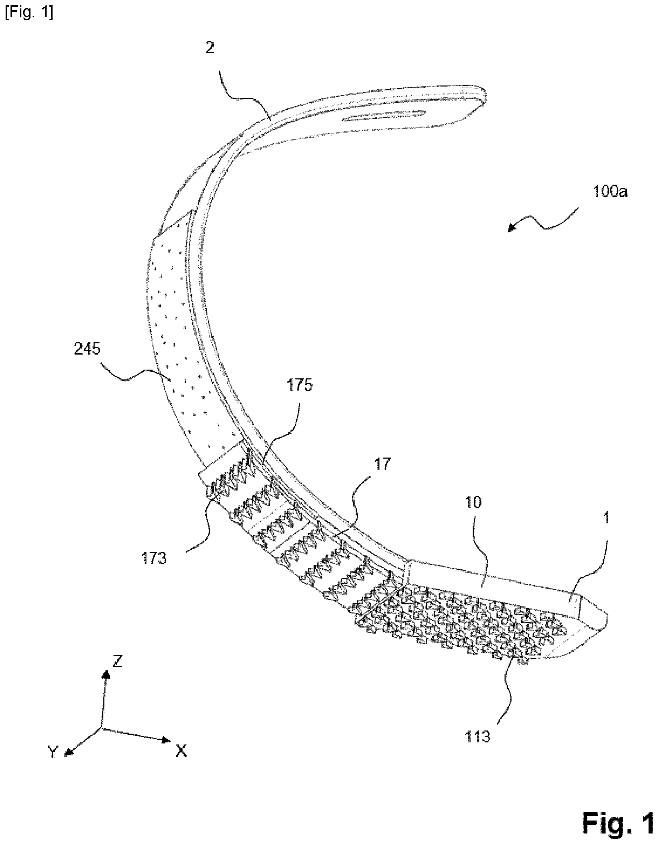

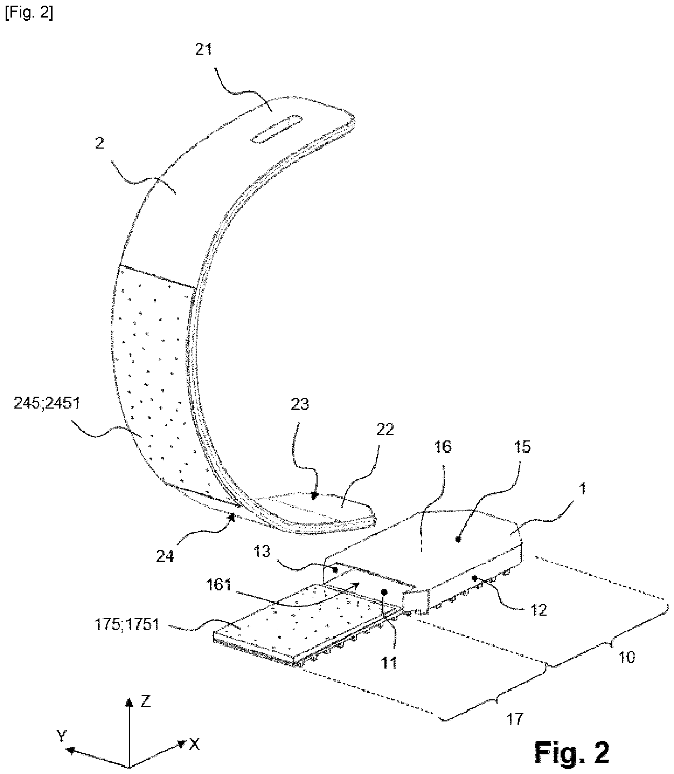

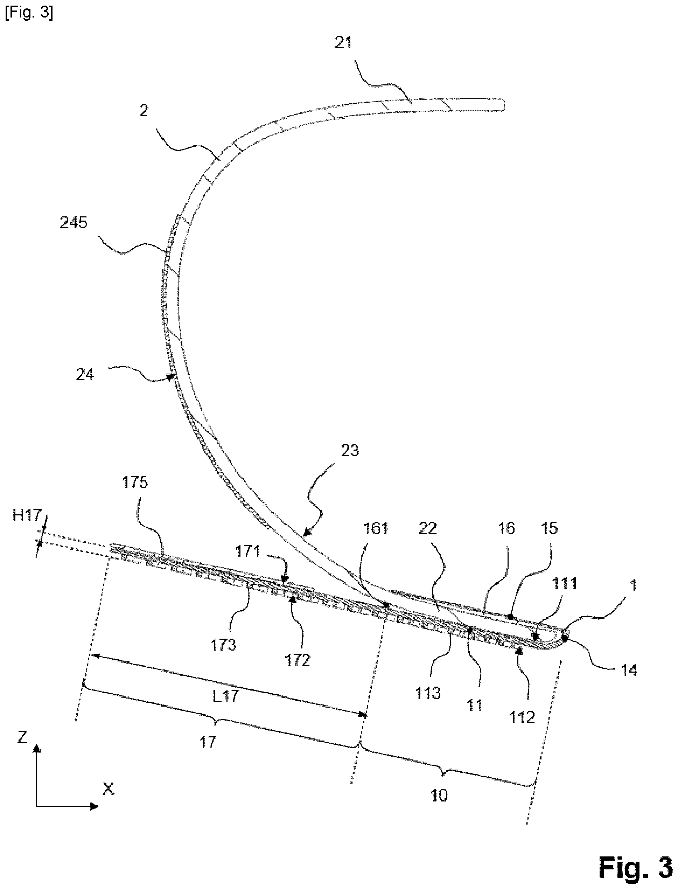

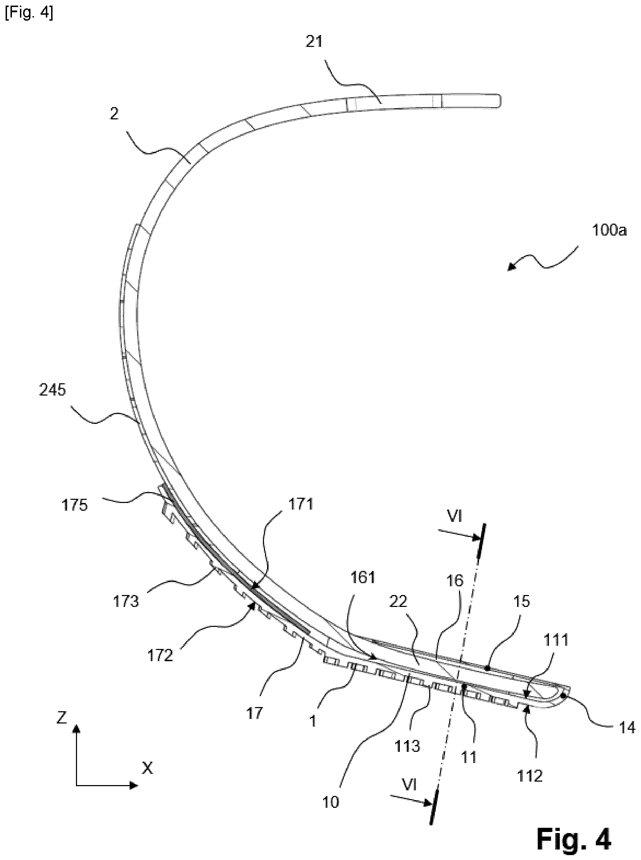

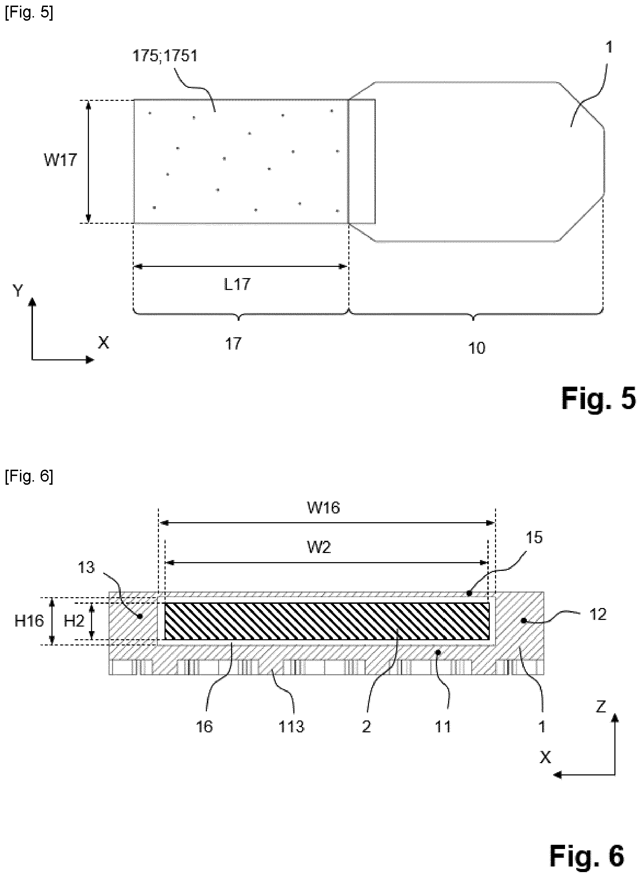

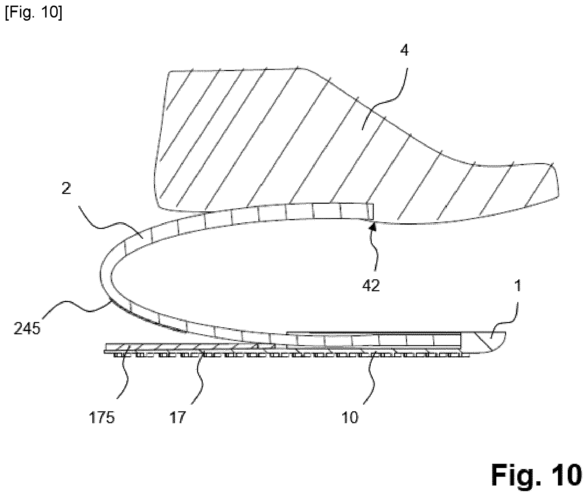

Les figures 1 à 7 illustrent la construction d'une semelle amovible 1 selon un premier mode de réalisation de l'invention. Cette semelle amovible 1 est destinée à se fixer sur une extrémité d'une lame flexible 2, réalisée, préférentiellement, en matériau composite. Ce peut être une lame en carbone pour fournir une très bonne restitution énergétique. Dans la description qui va suivre, cette lame flexible est une prothèse de jambe pour personne amputée d'un membre inférieur. Alternativement, comme représenté à la figure 10, cette lame flexible peut être un composant fixé sur la partie inférieure d'une chaussure, la lame flexible faisant alors l'interface entre la tige et le sol.

Une prothèse équipée d'une semelle 100a selon l'invention est représentée en perspective, vue de dessous, à la figure 1. La lame flexible, correspondant à la prothèse, est galbée. Elle comprend une première extrémité 21 destinée à se fixer sur l'extrémité du membre amputé et une deuxième extrémité, extrémité distale 22, opposée à la première extrémité. La deuxième extrémité 22 est sensiblement au droit de la première extrémité 21. La lame est caractérisée par une section sensiblement rectangulaire de largeur W2 et d'épaisseur H2. Dans cet exemple, la lame suit une trajectoire courbe convexe, s'étendant vers l'arrière du sportif. Alternativement, la lame peut suivre tout autre type trajectoire. La lame 2 comprend une face supérieure 23 et une face inférieure 24. Au sens de l'invention, la face inférieure 24 de la lame est la face faisant face au sol au niveau de l'extrémité distale 22. La face supérieure 23 de la lame est la face opposée à la face inférieure. La face supérieure 23 fait face au corps de l'utilisateur, au niveau de l'extrémité distale 22. Du fait que la lame forme sensiblement un arc, l'orientation des faces supérieure 23 et inférieure 24 varie. Ainsi, au niveau de la première extrémité 21, l'orientation est inversée. La face inférieure 24 fait face à l'utilisateur et la face supérieure 23 fait face au sol.

Le semelle amovible 1 selon l'invention est prévue pour s'insérer sur l'extrémité distale 22 de la lame flexible 2.

Le semelle amovible 1 comprend une partie antérieure 10 formée d'une paroi inférieure 11, d'une paroi latérale 12, d'une paroi médiale 13, d'une paroi frontale 14 et d'une paroi supérieure 15. L'ensemble de ces parois 11, 12, 13, 14, 15 délimite un logement 16 apte à recevoir une partie de l'extrémité distale 22 de la lame flexible 2. Le logement 16 présente ainsi une ouverture postérieure 161 délimitée par les bords postérieurs des parois inférieure 11, latérale 12, médiale 13 et supérieure 15 de la partie antérieure 10, l'ouverture postérieure 161 permettant l'insertion de l'extrémité distale 22 de la lame flexible 2 dans le logement 16.

Selon un mode de réalisation, le logement 16 est dimensionné de sorte que la section transversale du logement 16 correspond sensiblement à la section transversale de la partie distale 22 de la lame flexible 2. Ainsi, la largeur W16 et la hauteur H16 du logement 16 sont sensiblement égale ou très légèrement supérieure respectivement à la largeur W2 et l'épaisseur H2 de la partie distale 22 de la lame 2. Cela permet d'obtenir un bon maintien, transversal et vertical, de la semelle 1 sur la lame 2, une fois mise en place.

L'ensemble des parois inférieure 11, latérale 12, médiale 13, frontal 14 et supérieure 15 permettent de protéger l'extrémité distale 22 de la lame flexible 2. Les parois latérale 12, médiale 13, frontal 14 protègent plus particulièrement les chants de l'extrémité distale 2. Cette partie antérieure 10 de la semelle 1 permet donc de protéger la lame d'une détérioration résultant de la nature du terrain ou des intempéries, que ce soit une dégradation par abrasion via la roche, le bitume, les racines... ou une dégradation climatique via la neige, la pluie...

Dans cet exemple, la paroi supérieure 15 est une pièce distincte qui est fixée sur l'ensemble unitaire constitué des parois inférieure 11, latérale 12, médiale 13 et frontale 14. La paroi supérieure 15 est donc une pièce rapportée. La paroi supérieure 15 est, par exemple, collée ou soudée sur les parois latérale 12, médiale 13 et frontale 14. Cette construction en deux parties facilite la réalisation de ces deux pièces constitutives qui peuvent être obtenues avec un outillage simple et économique, à savoir des moules sans tiroirs, dit à « gaufre ». Les pièces peuvent être précises et sans défauts. Alternativement l'ensemble des parois 11, 12, 13, 14, 15 forme une pièce monobloc unitaire, ce qui permet de supprimer une étape d'assemblage postérieure.

Selon un mode de réalisation, une des parois, préférentiellement la paroi supérieure 15, comprend des ouvertures afin d'évacuer les éventuels corps étrangers (cailloux, boue...) qui se sont introduits dans le logement 16.

Dans cet exemple, une face inférieure 112 de la paroi inférieure 11, destinée à venir en contact avec le sol, est munie de crampons 113. Ces crampons 113 permettent une meilleure adhérence de la semelle avec le terrain sur lequel évolue le sportif.

Selon l'invention, la semelle amovible 1 comprend une extension postérieure 17 souple prolongeant la paroi inférieure 11 vers l'arrière. Dans cet exemple, l'extension postérieure 17 présente une largeur W17 sensiblement égale à la largeur W2 de la lame flexible 2 et s'étend sur une longueur L17 supérieure à la largeur W2 de la lame flexible 2 ou la largeur W16 du logement 16. Préférentiellement, la longueur L17 est 1,5 fois plus grande que la largeur W2 de la lame flexible 2 ou la largeur W16 du logement 16. Comme on le verra par la suite, cette extension postérieure 17 est destinée à recouvrir une partie de la face inférieure 24 de la lame 2. Ainsi, cette extension postérieure 17 va protéger davantage la lame, notamment dans sa zone arrière. Il s'avère que cette protection est utile dans certaine situation, par exemple, pour protéger la lame du contact avec le bord de marches lorsque l'utilisateur descend un escalier.

Au sens de l'invention, l'extension postérieure est souple lorsqu'elle peut se déformer pour s'adapter à la morphologie de la face inférieure 24 de la lame 2. L'extension postérieure peut ainsi se conformer pour reproduire sensiblement le profil de la face inférieure 24. Pour faciliter cette déformation, l'extension postérieure peut comprendre une charnière à l'interface entre la paroi inférieure 11 et l'extension postérieure 17. Ce peut être par une réduction de l'épaisseur à cette jonction.

L'extension postérieure 17 comprend une face supérieure 171 et une face inférieure 172. L'épaisseur H 17 de l'extension postérieure peut être constante ou, alternativement dégressive plus on s'éloigne de l'ouverture postérieure 161. Cette variation d'épaisseur augmente la souplesse de cette extension, ce qui permet une meilleure conformation au profil de la surface inférieure 24 de la lame 2.

Dans cet exemple, une face supérieure 111 de la paroi inférieure 11 et la face supérieure 171 de l'extension postérieure 17 se prolongent l'une l'autre, formant une surface continue.

Selon un mode de réalisation, la face inférieure 172 de l'extension postérieure 17 est munie de crampons 173. Cela permet une meilleure adhérence lors des foulées avec « attaque talon ».

La face supérieure 171 de l'extension postérieure 17 supporte un moyen d'accroche 175 apte à coopérer avec un moyen d'accroche complémentaire 245 disposé sur la face inférieure 24 de la lame flexible 2 de sorte solidariser la semelle 1 avec la lame flexible 2. Selon un mode de réalisation, le moyen d'accroche 175 est une surface 1751 composée de crochets et/ou de boucles et le moyen d'accroche complémentaire 245 est une surface complémentaire 2451 composée de boucles et/ou de crochets. Ainsi, ces moyens d'accroche sont agencés de sorte que les crochets coopèrent avec les boucles pour assurer la solidarisation des pièces. Ce type de moyens d'accroche est de type VELCRO^{®}. Ils permettent un assemblage et un désassemblage rapide et très facile à manipuler. De plus, l'accroche est suffisante pour maintenir la semelle sur la lame. Selon un mode de réalisation, la surface 1751 du moyen d'accroche 175 et la surface complémentaire 2451 du moyen d'accroche complémentaire 245 sont agencées et dimensionnées de sorte que le recouvrement entre les deux surfaces, une fois la semelle assemblée sur la lame, couvre au moins un carré dont la longueur d'un côté est sensiblement égale à la largeur de la lame W2 ou de l'extension postérieure W17. Avec une telle surface, l'adhérence est suffisante pour assurer un bon maintien relatif entre les deux pièces, la semelle et la lame.

D'autres types de moyens d'accroche pourraient être envisagés. Par exemple, cela pourrait être des boutons-pression.

En étant positionné sur la face supérieure 171 de l'extension postérieure 17, le moyen d'accroche 175 va être protégé par l'extension postérieure 17 lorsque la semelle est solidarisée avec la lame. Cette même extension postérieure 17 va également protéger le moyen d'accroche complémentaire 245 disposé sur la lame flexible 2.

Dans cet exemple, la semelle est majoritairement, voire à 100%, composée d'un même type de matériau ce qui permet de faciliter son recyclage.

Selon un mode de réalisation, les parois inférieure 11, latérale 12, médiale 13, frontale 14 et l'extension postérieure 17 sont en caoutchouc ce qui permet une bonne protection aux chocs et un bon maintien de la semelle sur la lame par enserrement. De plus, le caoutchouc présente de bonnes caractéristiques de souplesse ce qui permet d'assurer la flexibilité nécessaire à la conformation de l'extension postérieure 17 au profil de la face inférieure 24 de la lame flexible 2. La paroi supérieure 15 peut également être en caoutchouc.

Avantageusement, le caoutchouc est caractérisé par une dureté comprise entre 60 et 80 Shore A. Cet intervalle s'avère être un très bon compromis permettant, d'une part, d'assurer un bon maintien de la structure de la semelle 1 et donc une bonne tenue de la semelle sur la lame, mais aussi d'éviter l'affaissement ou le ballottement de la paroi inférieure 11 et, d'autre part, d'amortir le contact sol / lame, mais également d'apporter une protection de la lame, notamment aux chocs.

Selon un mode de réalisation, la paroi supérieure 15 est réalisée dans un matériau ayant des propriétés anisotropes, moins extensible transversalement que longitudinalement. Cela permet une bonne tenue latérale de la semelle par rapport à la lame, l'écartement des parois latérale 12 et médiale 13 étant contenu, tout en facilitant l'insertion de la semelle sur la lame du fait que la paroi pourra se déformer afin de suivre la cambrure de la surface supérieure de l'extrémité distale de la lame.

D'autres types de matériaux pourraient être envisagés pour réaliser ces parois.

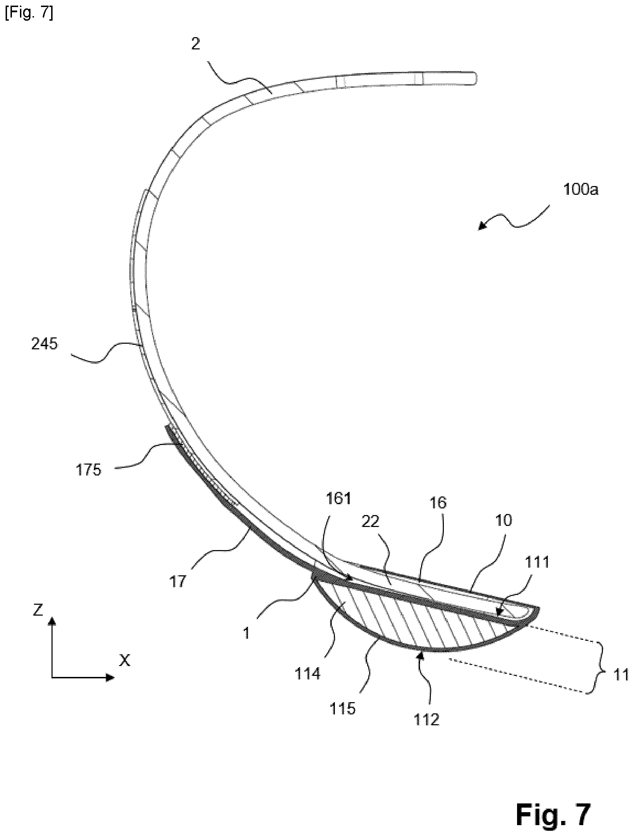

Selon un deuxième mode de réalisation, illustré à la figure 7, la paroi inférieure 11 comprend une couche d'amortissement 114 constituée d'un matériau amortissant comme, par exemple, un matériau à base de Ethylène-Acétate de Vinyle (EVA) ou une mousse PolyUréthane (PU). Dans ce cas, la paroi inférieure 11 comprend avantageusement une couche d'usure 115, en caoutchouc, recouvrant partiellement ou en totalité, la surface inférieure de la couche d'amortissement 114. Cette couche d'usure 115 est destinée à être en contact direct avec le sol et permet d'améliorer l'adhérence et de protéger la couche d'amortissement contre l'usure par abrasion/friction/perforation.

Toutes les constructions précédentes présentent l'intérêt d'être simple à utiliser, économique et robuste. Pour la mise en place de la semelle sur la lame, l'utilisateur insère l'extrémité distale 22 de la lame 2 dans le logement 16 de la semelle 2, à travers l'ouverture postérieure 161, jusqu'à butée contre la paroi frontale 14. Une fois l'extrémité distale 22 introduite dans ce logement 16, l'utilisateur ramène l'extension postérieure 17 contre la face inférieure 24 de la lame 2 de sorte à faire coopérer le moyen d'accroche 175 de l'extension postérieure 17 avec le moyen d'accroche complémentaire 245 de la lame 2. Cette coopération assure la solidarisation de la semelle avec la lame, d'une manière suffisante pour le maintien de la semelle lors d'une pratique sportive. Cette opération s'avère être très simple à réaliser. Pour retirer la semelle, il s'agit d'effectuer les opérations inverses. L'utilisateur agit sur l'extension postérieure 17 pour l'écarter de la face inférieure 24 de la lame ce qui a pour conséquence de désolidariser les moyens d'accroche 175, 245. Ensuite, il suffit d'agir sur la partie antérieure 10 de la semelle 1 pour désengager l'extrémité distale 22 du logement 16 de la semelle.

Grâce à la simplicité de cet assemblage, l'utilisateur peut facilement changer de semelle en fonction de l'environnement dans lequel il évolue ou suite à l'usure / la détérioration de la semelle. C'est un avantage indéniable lorsque l'utilisateur court dans un terrain changeant, comme la montagne, où il peut être nécessaire d'adapter l'adhérence de la semelle.

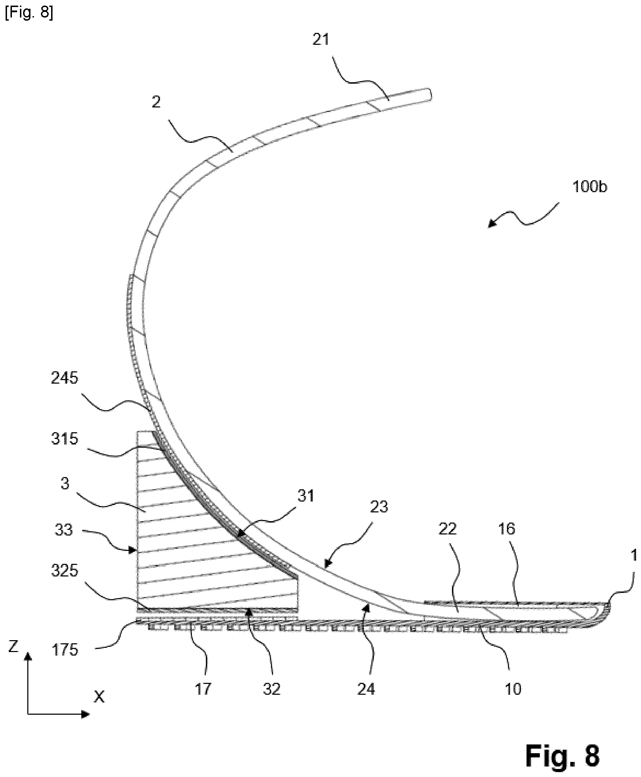

Toutes les constructions précédentes présentent également un intérêt concernant la possibilité d'ajuster la stabilité de portage. En effet, ces solutions sont compatibles avec la mise en place d'une cale intercalaire 3 entre l'extension postérieure 17 de la semelle 1 et la lame 2. Cette configuration est illustrée à la figure 8. Dans ce troisième mode de réalisation, la cale intercalaire 3 se présente sous une forme d'un prisme triangulaire comprenant une face supérieure 31, une face inférieure 32 et face postérieure 33 pour une largeur W3. La face supérieure 31 porte un moyen d'accroche 315 sensiblement analogue au moyen d'accroche 175 de l'extension postérieure 17. Le moyen d'accroche 315 est apte à coopérer avec le moyen d'accroche complémentaire 245 de la lame 2. La face inférieure 32 porte un moyen d'accroche complémentaire 325 sensiblement analogue au moyen d'accroche complémentaire 245 de la lame 2. Le moyen d'accroche complémentaire 315 est apte à coopérer avec le moyen d'accroche 175 de l'extension postérieure 17. La hauteur de la face postérieure 33 définit la hauteur de la cale intercalaire 3. Elle permet d'ajuster la surface de contact de la semelle avec le sol. On peut ainsi augmenter la surface de contact au sol en positionnant la surface inférieure 172 de l'extension postérieure 17 plus proche du sol. La hauteur de la cale permet d'ajuster l'inclinaison, le « drop » de la semelle. La stabilité est alors nettement améliorée, ce qui peut être recherché en phase statique ou lors de descente. Cette stabilité apporte davantage de sécurité et permet à l'utilisateur de se relâcher, de se reposer et de récupérer de l'énergie. La cale renvoie également une information d'appui talon qui améliore la proprioception et les sensations de l'utilisateur. Cette amélioration de la performance est marquée sur le plat et en descente, principalement en terrain accidenté.

Dans cet exemple, l'extension postérieure 17 de la semelle amovible 1 est apte à recouvrir la surface inférieure 32 de la cale intercalaire 3 au niveau du talon. Ainsi, la cale n'a pas de contact direct avec le sol du fait que l'extension postérieure de la semelle amovible s'intercale entre la cale et le sol. Cela permet de protéger la cale et d'éviter alors qu'elle s'endommage avec les aspérités du sol ou les frottements avec celui-ci : cailloux, rochers, racines, bitume... Cela permet aussi d'avoir une construction de cale simple et économique, sans avoir besoin d'ajouter une couche d'usure, par exemple une couche de caoutchouc, au niveau du talon.

La largeur de la cale W3 est sensiblement égale à la largeur de la lame W2 ou la largeur W17 de l'extension postérieure 17.

Par ailleurs, avantageusement, la cale est réalisée avec un matériau amortissant pour procurer plus de confort. Un tel matériau peut être un matériau à base de Ethylène-Acétate de Vinyle (EVA) ou une mousse PolyUréthane (PU). La cale peut comprendre des parties constituées d'un matériau autre qu'un matériau amortissant.

Là encore, la mise en place de la cale est simple et intuitive. Il suffit d'intercaler la cale entre l'extension postérieure 17 et la lame 2, en faisant coopérer les moyens d'accroches respectifs correspondant 315/245 et 325/175.

Un mode de réalisation de l'invention porte ainsi sur un ensemble comprenant 100b :
- une lame flexible présentant une extrémité distale 22 et une face inférieure 24, la face inférieure portant des moyens d'accroche complémentaires 245 dans une zone arrière,
- une semelle amovible telle que définie dans l'une des revendications précédentes, la semelle amovible étant destinée à s'emboiter sur l'extrémité distale 22 de la lame flexible, les moyens d'accroche 175 de la semelle amovible étant apte à coopérer avec les moyens d'accroche complémentaires 245 de la lame flexible.
- une cale 3 intercalée entre la lame flexible 2 et l'extension postérieure 17 de la semelle amovible, la cale présentant une face inférieure 32 munie de moyens d'accroche complémentaires 325 apte à coopérer avec les moyens d'accroche 175 de la face supérieure 171 de l'extension postérieure 17 de la semelle amovible, la cale présentant une face supérieure 31 munie de moyens d'accroche 315 apte à coopérer avec moyens d'accroche complémentaires 245 de la face inférieure 24 de la lame flexible.

Des cales plus élaborées ou en multi-matériaux pourraient être envisagées.

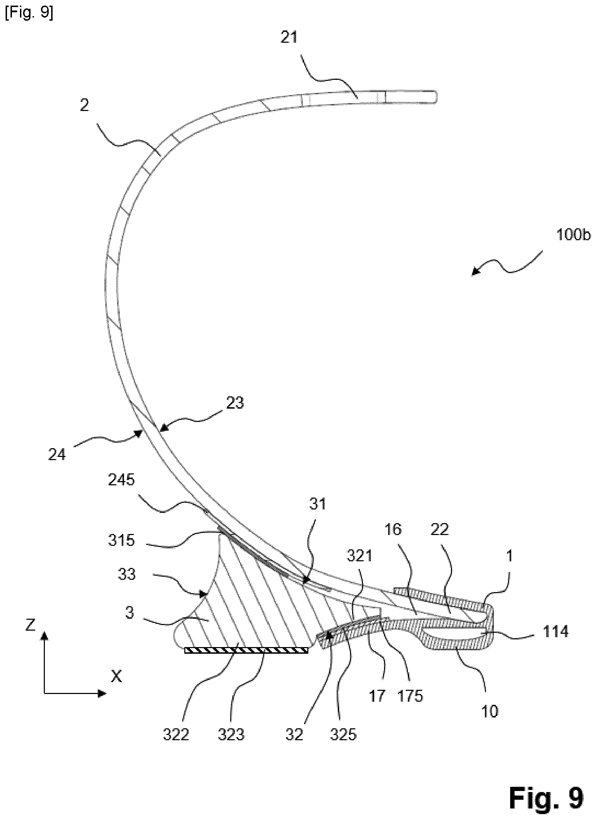

La figure 9 illustre un quatrième mode de réalisation de la cale intermédiaire 3. Comme illustré précédemment, la cale comprend une face supérieure 31, une face inférieure 32. La face supérieure 31 porte un moyen d'accroche 315 sensiblement analogue au moyen d'accroche 175 de l'extension postérieure 17. Le moyen d'accroche 315 est apte à coopérer avec le moyen d'accroche complémentaire 245 de la lame 2. La face inférieure 32 porte un moyen d'accroche complémentaire 325 sensiblement analogue au moyen d'accroche complémentaire 245 de la lame 2. Le moyen d'accroche complémentaire 315 est apte à coopérer avec le moyen d'accroche 175 de l'extension postérieure 17. Dans cet exemple, la face inférieure 32 est une surface gauche formant un arc 321 représentant une partie de la voûte plantaire et un talon 322 sensiblement plat dans sa partie postérieure. Ici, le moyen d'accroche 175 de l'extension postérieure 17 est fixée au niveau de l'arc 321. La cale est préférentiellement réalisée avec un matériau amortissant. Au niveau du talon 322, la surface inférieure est avantageusement recouverte d'une couche d'usure 323, formant des crampons, réalisée préférentiellement en caoutchouc. Alternativement, l'extension postérieure est apte à recouvrir la surface inférieure au niveau du talon, comme décrit dans le mode de réalisation précédent.

Ce quatrième mode de réalisation permet d'envisager l'utilisation de cales à différentes pointures, longueur orteil-talon. On peut ainsi prévoir une pointure proche de la pointure de l'autre pied valide, dans le cas d'une personne amputée d'un seul membre.

Les exemples précédents décrivent une semelle pour lame orthopédique. La solution peut aussi s'appliquer à des chaussures pour personnes valides. La figure 10 illustre une telle transposition. La lame 2 est fixée sur une face inférieure 42 d'une tige 4. La semelle 1 vient alors se mettre en place sur l'extrémité 22 de la lame 2 comme décrit précédemment.

L'invention n'est pas limitée aux modes de réalisation précédemment décrits. Il est également possible de combiner ces modes de réalisation. L'invention s'étend à tous les modes de réalisation couverts par les revendications annexées.

### Références

- 1.: Semelle amovible
10. Partie antérieure
11. Paroi inférieure
111. Face supérieure
112. Face inférieure
113. Crampons
114. Couche d'amortissement
115. Couche d'usure
12. Paroi latérale
13. Paroi médiale
14. Paroi frontale
15. Paroi supérieure
16. Logement
161. Ouverture postérieure
17. Extension postérieure
171. Face supérieure
172. Face inférieure
173. Crampons
175. Moyen d'accroche
1751. Surface
- 2.: Lame flexible
21. Première extrémité
22. Deuxième extrémité
23. Face supérieure
24. Face inférieure
245. Moyen d'accroche complémentaire
2451. Surface complémentaire
- 3.: Cale intercalaire
31. Face supérieure
315. Moyen d'accroche
32. Face inférieure
321. Arc
322. Talon
323. Couche d'usure
325. Moyen d'accroche complémentaire
33. Face postérieure
- 4.: Tige
42. Face inférieure

## Revendications

1. Semelle amovible (1) composée d'une partie antérieure (10) comprenant un logement (16) destiné à recevoir une extrémité distale (22) d'une lame flexible (2), le logement étant délimité par une paroi inférieure (11), une paroi latérale (12), une paroi médiale (13), une paroi frontale (14) et une paroi supérieure (15), le logement présentant ainsi une ouverture postérieure (161) délimitée par les parois inférieure, latérale, médiale et supérieure de la partie antérieure, l'ouverture postérieure permettant l'insertion de l'extrémité distale de la lame flexible dans le logement,
**caractérisée en ce que**
la semelle amovible comprend une extension postérieure (17) souple prolongeant la paroi inférieure vers l'arrière, l'extension postérieure présentant une face supérieure (171) portant un moyen d'accroche (175) apte à coopérer avec un moyen d'accroche complémentaire (245) disposé sur une face inférieure (24) de la lame flexible.

2. Semelle amovible (1) selon la revendication 1 **caractérisée en ce que** la face supérieure (111) de la paroi inférieure (1) et la face supérieure (171) de l'extension postérieure (17) se prolongent l'une l'autre, formant une surface continue.

3. Semelle amovible (1) selon l'une des revendications précédentes **caractérisée en ce que** le moyen d'accroche de la face supérieure de l'extension postérieure sont composés de crochets et/ou de boucles.

4. Semelle amovible (1) selon l'une des revendications précédentes **caractérisée en ce que** la paroi inférieure (11) et l'extension postérieure (17) comprennent des faces inférieures (112, 172) constituées de caoutchouc.

5. Semelle amovible (1) selon l'une des revendications précédentes **caractérisée en ce que** les parois latérale (12), médiale (13) et frontale (14) sont en caoutchouc.

6. Semelle amovible (1) selon l'une des deux revendications précédentes **caractérisée en ce que** le caoutchouc est **caractérisé par** une dureté comprise entre 60 et 80 Shore A.

7. Semelle amovible (1) selon l'une des revendications précédentes **caractérisée en ce que** la paroi inférieure (11) comprend au moins une portion (114) constituée d'un matériau à base de Ethylène-Acétate de Vinyle (EVA) ou mousse PolyUréthane (PU).

8. Semelle amovible (1) selon l'une des revendications précédentes **caractérisée en ce que** la paroi supérieure (15) est une pièce rapportée.

9. Semelle amovible (1) selon l'une des revendications précédentes **caractérisée en ce que** la paroi supérieure est réalisée dans un matériau ayant des propriétés anisotropes, moins extensible transversalement que longitudinalement.

10. Semelle amovible (1) selon l'une des revendications précédentes **caractérisée** en l'extension postérieure (17) présente une largeur (W17) sensiblement égale à la largeur (W2) de la lame flexible (2) et s'étend sur une longueur (L17) supérieure à la largeur de la lame flexible.

11. Semelle amovible (1) selon l'une des revendications précédentes **caractérisée en ce que** la largeur (W16) et la hauteur (H16) du logement (16) sont sensiblement égale ou très légèrement supérieure respectivement à la largeur (W2) et l'épaisseur (H2) de la partie distale (22) de la lame flexible (2).

12. Ensemble (100a) comprenant :
- une lame flexible présentant une extrémité distale (22) et une face inférieure (24), la face inférieure portant des moyens d'accroche complémentaires (245) dans une zone arrière,
- une semelle amovible telle que définie dans l'une des revendications précédentes, la semelle amovible étant destinée à s'emboiter sur l'extrémité distale (22) de la lame flexible, les moyens d'accroche (175) de la semelle amovible étant apte à coopérer avec les moyens d'accroche complémentaires (245) de la lame flexible.

13. Ensemble (100b) selon la revendication précédente **caractérisé en ce qu'**il comprend en outre, une cale (3) intercalée entre la lame flexible (2) et l'extension postérieure (17) de la semelle amovible, la cale présentant une face inférieure (32) munie de moyens d'accroche complémentaires (325) apte à coopérer avec les moyens d'accroche (175) de la face supérieure (171) de l'extension postérieure (17) de la semelle amovible, la cale présentant une face supérieure (31) munie de moyens d'accroche (315) apte à coopérer avec moyens d'accroche complémentaires (245) de la face inférieure (24) de la lame flexible.

14. Ensemble (100b) selon la revendication précédente **caractérisé en ce que** la cale comprend au moins une portion constituée d'un matériau à base de Ethylène-Acétate de Vinyle (EVA) ou mousse PolyUréthane (PU).

15. Ensemble (100b) selon l'une des deux revendications précédentes **caractérisé en ce que** l'extension postérieure (17) de la semelle amovible (1) est apte à recouvrir la surface inférieure (32) de la cale intercalaire (3) au niveau du talon

## Patentansprüche

1. Abnehmbare Sohle (1), die aus einem vorderen Teil (10) zusammengesetzt ist, der eine Aufnahme (16) umfasst, die dazu bestimmt ist, ein distales Ende (22) einer Blattfeder (2) aufzunehmen, wobei die Aufnahme durch eine untere Wand (11), eine seitliche Wand (12), eine mediale Wand (13), eine frontale Wand (14) und eine obere Wand (15) begrenzt wird, wobei die Aufnahme somit eine rückwärtige Öffnung (161) aufweist, die durch die unteren, seitlichen, medialen und oberen Wände des vorderen Teils begrenzt wird, wobei die rückwärtige Öffnung das Einführen des distalen Endes der Blattfeder in die Aufnahme ermöglicht,
**dadurch gekennzeichnet, dass** die abnehmbare Sohle eine elastische rückwärtige Erweiterung (17) umfasst, welche die untere Wand nach hinten verlängert, wobei die rückwärtige Erweiterung eine Oberseite (171) aufweist, die ein Klettmittel (175) trägt, das geeignet ist, mit einem komplementären Klettmittel (245) zusammenzuwirken, das auf einer Unterseite (24) der Blattfeder angeordnet ist.

2. Abnehmbare Sohle (1) nach Anspruch 1, **dadurch gekennzeichnet, dass** die Oberseite (111) der unteren Wand (1) und die Oberseite (171) der rückwärtigen Erweiterung (17) einander verlängern, wobei sie eine durchgehende Fläche bilden.

3. Abnehmbare Sohle (1) nach einem der vorhergehenden Ansprüche, **dadurch gekennzeichnet, dass** das Klettmittel der Oberseite der rückwärtigen Erweiterung aus Haken und/oder Schlaufen zusammengesetzt ist.

4. Abnehmbare Sohle (1) nach einem der vorhergehenden Ansprüche, **dadurch gekennzeichnet, dass** die untere Wand (11) und die rückwärtige Erweiterung (17) Unterseiten (112, 172) umfassen, die aus Kautschuk bestehen.

5. Abnehmbare Sohle (1) nach einem der vorhergehenden Ansprüche, **dadurch gekennzeichnet, dass** die seitlichen (12), medialen (13) und frontalen (14) Wände aus Kautschuk sind.

6. Abnehmbare Sohle (1) nach einem der beiden vorhergehenden Ansprüche, **dadurch gekennzeichnet, dass** der Kautschuk durch eine Härte zwischen 60 und 80 Shore A gekennzeichnet ist.

7. Abnehmbare Sohle (1) nach einem der vorhergehenden Ansprüche, **dadurch gekennzeichnet, dass** die untere Wand (11) mindestens einen Abschnitt (114) umfasst, der aus einem Material auf Basis von Ethylen-Vinylacetat (EVA) oder Polyurethan-Schaum (PU) besteht.

8. Abnehmbare Sohle (1) nach einem der vorhergehenden Ansprüche, **dadurch gekennzeichnet, dass** die obere Wand (15) ein angesetztes Teil ist.

9. Abnehmbare Sohle (1) nach einem der vorhergehenden Ansprüche, **dadurch gekennzeichnet, dass** die obere Wand aus einem Material ausgeführt ist, das anisotrope Eigenschaften hat, quer weniger dehnbar als längs ist.

10. Abnehmbare Sohle (1) nach einem der vorhergehenden Ansprüche, gekennzeichnet, dass die rückwärtige Erweiterung (17) eine Breite (W17) aufweist, die im Wesentlichen gleich der Breite (W2) der Blattfeder (2) ist, und sich über eine Länge (L17) erstreckt, die größer als die Breite der Blattfeder ist.

11. Abnehmbare Sohle (1) nach einem der vorhergehenden Ansprüche, **dadurch gekennzeichnet, dass** die Breite (W16) und die Höhe (H16) der Aufnahme (16) im Wesentlichen gleich der oder ganz geringfügig größer als die Breite (W2) und die Dicke (H2) des distalen Teils (22) der Blattfeder (2) sind.

12. Anordnung(100a), umfassend:
- eine Blattfeder, die ein distales Ende (22) und eine Unterseite (24) aufweist, wobei die Unterseite komplementäre Klettmittel (245) in einem hinteren Bereich trägt,
- eine abnehmbare Sohle wie in einem der vorhergehenden Ansprüche definiert, wobei die abnehmbare Sohle dazu bestimmt ist, auf das distale Ende (22) der Blattfeder gesteckt zu werden, wobei die Klettmittel (175) der abnehmbaren Sohle geeignet sind, mit den komplementären Klettmitteln (245) der Blattfeder zusammenzuwirken.

13. Anordnung (100b) nach dem vorhergehenden Anspruch, **dadurch gekennzeichnet, dass** sie ferner einen Keil (3) umfasst, der zwischen der Blattfeder (2) und der rückwärtigen Erweiterung (17) der abnehmbaren Sohle eingelegt ist, wobei der Keil eine Unterseite (32) aufweist, die mit komplementären Klettmitteln (325) versehen ist, die geeignet sind, mit den Klettmitteln (175) der Oberseite (171) der rückwärtigen Erweiterung (17) der abnehmbaren Sohle zusammenzuwirken, wobei der Keil eine Oberseite (31) aufweist, die mit Klettmitteln (315) versehen ist, die geeignet sind, mit komplementären Klettmitteln (245) der Unterseite (24) der Blattfeder zusammenzuwirken.

14. Anordnung (100b) nach dem vorhergehenden Anspruch, **dadurch gekennzeichnet, dass** der Keil mindestens einen Abschnitt umfasst, der aus einem Material auf Basis von Ethylen-Vinylacetat (EVA) oder Polyurethan-Schaum (PU) besteht.

15. Anordnung (100b) nach einem der beiden vorhergehenden Ansprüche, **dadurch gekennzeichnet, dass** die rückwärtige Erweiterung (17) der abnehmbaren Sohle (1) geeignet ist, die untere Fläche (32) des Einlegekeils (3) an der Ferse zu bedecken.

## Claims

1. Removable sole plate (1) composed of an anterior part (10) comprising a housing (16) intended to receive a distal end (22) of a flexible blade (2), the housing being delimited by a bottom wall (11), a lateral wall (12), a medial wall (13), a front-end wall (14) and a top wall (15), the housing thus having a posterior aperture (161) delimited by the bottom, lateral, medial and top walls of the anterior part, the posterior aperture allowing the insertion of the distal end of the flexible blade into the housing,
**characterized in that**
the removable sole plate comprises a flexible posterior extension (17) prolonging the bottom wall towards the rear, the posterior extension having a top face (171) bearing an attachment means (175) capable of cooperating with a complementary attachment means (245) disposed on a bottom face (24) of the flexible blade.

2. Removable sole plate (1) according to Claim 1, **characterized in that** the top face (111) of the bottom wall (1) and the top face (171) of the posterior extension (17) prolong one another, forming a continuous surface.

3. Removable sole plate (1) according to either of the preceding claims, **characterized in that** the attachment means of the top face of the posterior extension is composed of hooks and/or loops.

4. Removable sole plate (1) according to one of the preceding claims, **characterized in that** the bottom wall (11) and the posterior extension (17) comprise bottom faces (112, 172) composed of rubber.

5. Removable sole plate (1) according to one of the preceding claims, **characterized in that** the lateral (12), medial (13) and front-end (14) walls are made of rubber.

6. Removable sole plate (1) according to one of the two preceding claims, **characterized in that** the rubber is **characterized by** a hardness of between 60 and 80 Shore A.

7. Removable sole plate (1) according to one of the preceding claims, **characterized in that** the bottom wall (11) comprises at least one portion (114) composed of a material based on ethylene vinyl acetate (EVA) or polyurethane (PU) foam.

8. Removable sole plate (1) according to one of the preceding claims, **characterized in that** the top wall (15) is an added part.

9. Removable sole plate (1) according to one of the preceding claims, **characterized in that** the top wall is produced in a material having anisotropic properties, less extensible transversely than longitudinally.

10. Removable sole plate (1) according to one of the preceding claims, **characterized in that** the posterior extension (17) has a width (W17) substantially equal to the width (W2) of the flexible blade (2) and extends over a length (L17) greater than the width of the flexible blade.

11. Removable sole plate (1) according to one of the preceding claims, **characterized in that** the width (W16) and the height (H16) of the housing (16) are substantially equal to or very slightly greater respectively than the width (W2) and the thickness (H2) of the distal part (22) of the flexible blade (2).

12. Assembly (100a) comprising:
- a flexible blade having a distal end (22) and a bottom face (24), the bottom face bearing complementary attachment means (245) in a rear zone,
- a removable sole plate as defined in one of the preceding claims, the removable sole plate being intended to be fitted onto the distal end (22) of the flexible blade, the attachment means (175) of the removable sole plate being capable of cooperating with the complementary attachment means (245) of the flexible blade.

13. Assembly (100b) according to the preceding claim, **characterized in that** it further comprises a wedge (3) inserted between the flexible blade (2) and the posterior extension (17) of the removable sole plate, the wedge having a bottom face (32) provided with complementary attachment means (325) capable of cooperating with the attachment means (175) of the top face (171) of the posterior extension (17) of the removable sole plate, the wedge having a top face (31) provided with attachment means (315) capable of cooperating with complementary attachment means (245) of the bottom face (24) of the flexible blade.

14. Assembly (100b) according to the preceding claim, **characterized in that** the wedge comprises at least one portion composed of a material based on ethylene vinyl acetate (EVA) or polyurethane (PU) foam.

15. Assembly (100b) according to one of the two preceding claims, **characterized in that** the posterior extension (17) of the removable sole plate (1) is able to cover the bottom surface (32) of the insert wedge (3) at the heel.
